# EUROPEAN PATENT APPLICATION

(11) **EP 1 134 291 A1**
(43) Date of publication of application: **19.09.2001**
(21) Application number: 99972692.0
(22) Date of filing: 25.11.1999
(51) Int. Cl.: C12Q 1/66, C12Q 1/06, C12M 1/34

(54) **METHOD FOR COUNTING LIVING CELLS**

(30) Priority: 25.11.1998 JP 33397998
(71) Applicant: KIKKOMAN CORPORATION, Noda-shi, Chiba 278-8601 (JP)
(72) Inventor: HATTORI, Noriaki, Noda-shi, Chiba 278-8601 (JP); HARADA, Yasuhiro, Noda-shi, Chiba 278-8601 (JP); YAJITATE, Keiko, Noda-shi, Chiba 278-8601 (JP); MURAKAMI, Seiji, Noda-shi, Chiba 278-8601 (JP)
(74) Representative: Brasnett, Adrian Hugh
(86) International application number: JP9906582
(87) International publication number: WO0031292

(57) **Abstract**

The present invention provides a method for rapidly and sensitively counting living cells in a sample by serially diluting the sample containing living cells with a growth medium for living cells, culturing the resulting dilution, and then measuring an intracellular ATP level in the dilution using a luciferin-luciferase luminescent reaction; an assay kit used for the above method; and a living cell counting device for carrying out the above method.

## Description

### TECHNICAL FIELD

The present invention relates to a method for rapidly and sensitively counting living cells present in a sample, an assay reagent kit, and a living cell counting device.

### BACKGROUND ART

In various industrial fields, clinical medicine, basic research and the like, cell counting is broadly carried out for determining the number of living cells in a sample liquid. Particularly, in the food, pharmaceutical and cosmetic industries, it is very important to control the number of living cells, including microorganisms, present in industrial water, raw materials, intermediates or end products. In this case, a rapid determination is required rather than more perfect cell counting.

To determine the number of living cells in a sample, in general, a known plate culture method is conventionally carried out, in which living cells in the sample are cultured on a standard agar medium for a long time, followed by the detection of colonies formed thereon. The sample may be used for the culture as such or after trapping the living cells on a simple filter. See "Guidelines for Food Sanitation Inspection - Microorganisms" by Environmental Health Bureau, Ministry of Health and Welfare (1990) pp.70-77. In addition, other known methods for determining the number of living cells in a sample include the most probable number method (also called MPN method or Serial dilution method), which is now employed in Japan as an official method for analyzing the quality of pharmaceuticals and cosmetics etc. (The Japanese Pharmacopoeia, Thirteenth Edition, published by Hirokawa Publishing Co. (1996) p.B-403 to p.B-421) In the most probable number method, the number of living cells is determined by serially diluting a sample; inoculating an aliquot of each dilution into a liquid medium, followed by culture for a long time; determining the presence of living cells grown in the medium by its turbidity; and then calculating the number of living cells in the sample as the most probable number, based on a statistical probability theory.

In the plate culture method, the number of living cells is determined by applying a sample onto a solid plate medium such as agar medium, culturing it for about 2 to 7 days, and then counting colonies grown on the plate medium. Accordingly, the plate culture method has a disadvantage of permitting no rapid determination. The most probable number method also has a disadvantage of requiring a long culture period because the presence of living cells grown in the medium is determined by turbidimetry.

Recently, a luminescent reaction based on a luciferin-luciferase reaction is utilized for counting living cells (e.g., De Luca, M. & McElroy, W.D. (1978) Methods Enzymol. 57:3-15; Stanley, P.E. (1986) Methods Enzymol. 133:14-22). In the cell counting method based on the luciferin-luciferase luminescent reaction, the number of living cells is determined by extracting ATP from living cells, allowing the extracted ATP to produce luminescence through the luciferin-luciferase luminescent reaction, measuring an ATP level from the intensity of luminescence, and then calculating the number of living cells from the measured ATP level and an ATP level per living cell.

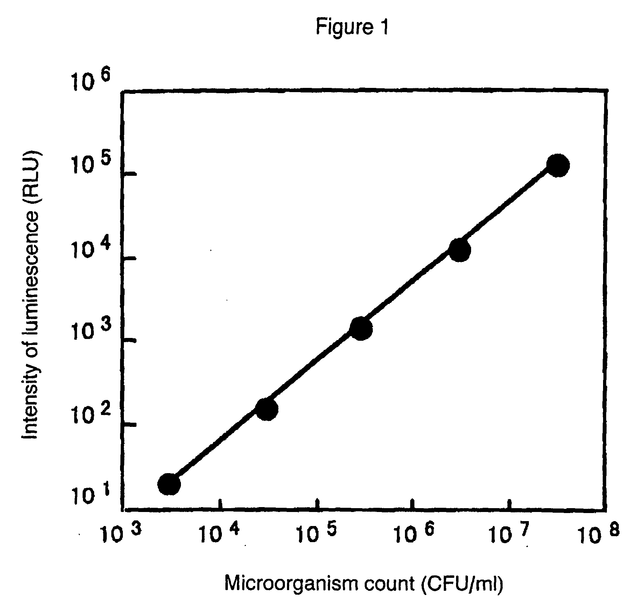

By way of example for cell counting using a bioluminescent reagent through the luciferin-luciferase luminescent reaction, Figure 1 shows the calibration curve prepared using CheckLite 250 Plus (Kikkoman Corp.) that converts the luminescence intensity into the number of *Escherichia coli* cells. As shown in Figure 1, the lower detection limit for *Escherichia coli* is 10³ CFU (unit of living cell)/ml. On the other hand, the ATP content per bacterial cell is reported to be almost constant among bacterial cell types (Kodaka et al., Japanese Journal of Food Microbiology, 13:29-34 (1996)). When the bioluminescence method is applied for bacterial cells in general, it is therefore possible to determine the number of living cells using the calibration curve mentioned above (i.e., Figure 1), but the detection limit in this case is also 10³ CFU/ml. Namely, the above method involves a problem that it is theoretically impossible to determine the number of living cells below the lower detection limit.

Further, when used for cell counting, the above method involves another problem that if ATP other than intracellular ATP (hereinafter, background ATP) is present at a high level in a sample, the intracellular ATP is buried in the background ATP, leading to a decrease in detectability. The decomposition of background ATP may be carried out using a reagent containing ATPase as an active ingredient. However, such a treatment cannot overcome the problem because it might results in insufficient decomposition of ATP.

With regard to a sample below the lower detection limit or containing background ATP at a high level, such a sample may be cultured for a short time to grow living cells to a detectable level. This makes the living cells detectable, but it is still impossible to determine the number of living cells in the sample.

The object of the present invention is to provide a method for counting living cells present in a sample with high accuracy in a short time, an assay reagent kit used for the method, and a living cell counting device for carrying out the method.

### DISCLOSURE OF THE INVENTION

Our research efforts were directed to overcoming the above problems, and we have found that the number of living cells in a sample can be determined with high accuracy in a short time by serially diluting the sample with a growth medium for living cells, culturing the resulting dilution, and then determining the presence of living cells in the cultured dilution using the luciferin-luciferase luminescent reaction, thereby finally completing the invention.

Namely, the present invention provides the following embodiments (1) to (7).
(1) A method for counting living cells in a sample, which comprises the following steps (i) to (iv):
   (i) a first step for serially diluting the sample with a diluent solution;
   (ii) a second step for culturing each dilution from the first step;
   (iii) a third step for detecting intracellular ATP in each of the cultured dilutions from the second step; and
   (iv) a fourth step for calculating the number of living cells in the sample, based on the results of intracellular ATP detection from the third step.
(2) The method according to (1) above, wherein the diluent solution used in the first step is a growth medium for living cells.
(3) The method according to (1) above, wherein the diluent solution used in the first step is a growth medium for living cells which has been subjected to ATP decomposition.
(4) The method according to (1) above, wherein ATP present in the dilution, excluding the intracellular ATP, is decomposed during or after the culture in the second step.
(5) The method according to (1) above, wherein the intracellular ATP is detected in the third step using a luciferin-luciferase luminescent reaction.
(6) An assay reagent kit used for the method according to (1) above, which comprises the following components (a) to (c):
   (a) a diluent solution,
   (b) an ATP-extracting reagent, and
   (c) an ATP-detecting reagent.
(7) A living cell counting device used for the method according to (1) above, which comprises a device for sample dilution, a culture device for cell growth and a device for ATP detection.
(8) The living cell counting device according to (7) above, wherein the device for ATP detection measures the intensity of luminescence produced through a luciferin-luciferase luminescent reaction.

This specification includes part or all of the contents as disclosed in the specification and/or drawings of Japanese Patent Application No. 10-333979, which is a priority document of the present application.

### BRIEF DESCRIPTION OF DRAWING

Figure 1 shows the calibration curve that was mentioned in Background Art and used in comparative method 2 of Examples 1, 2 and 3.

### BEST MODE FOR CARRYING OUT THE INVENTION

As used herein, the term "living cell" means any cell capable of growing. Such living cells include, but are not limited to, animal cells, plant cells and microorganisms (e.g., bacteria, yeast, filamentous fungi), and may be selected depending on the application purpose of the method according to the present invention. They may be used alone or by mixture.

A "sample" may be any sample suspected of containing the above living cells, including foods and drinks, pharmaceuticals, cosmetics, sea water, river water, industrial water, sewage, soil, urine, feces, blood, sputum, pus, and living cell cultures. The sample is used in liquid form for assay. When the sample contains solids or is viscous, it is desirably suspended in an appropriate solvent to give a solution sample. Such a solvent includes distilled water, physiological saline, phosphate buffer, Tris buffer and sodium acetate buffer.

In the first step of the present invention, a collected sample is subjected to serial dilution using a diluent solution. Illustrative examples of a diluent solution include, in addition to the growth medium for living cells mentioned above, distilled water, physiological saline, phosphate buffer, Tris buffer and sodium acetate buffer. In particular, the growth medium for living cells to be assayed is preferably used as a diluent solution. As used herein, the term "growth medium for living cells" means a solution that permits the proliferation and growth of living cells and that contains nutrient components necessary for their proliferation and growth. Any growth medium suitable for target living cells may be selected as a growth medium for living cells used in the present invention. Once target living cells are specified, those skilled in the art will readily select a suitable growth medium. For example, a suitable growth medium may be Dulbecco's MEM medium for animal cell growth, nutrient broth medium for bacterial cell growth, and dextrose peptone broth for yeast cell growth.

The serial dilution means a series of dilution cycles repeated in a manner that a sample is diluted with a diluent solution to give a first dilution, which is then similarly diluted with a diluent solution to give a second dilution. The serial dilution can provide a stepwise increase in dilution factor. Further, any dilution factor may be selected without any particular limitation. However, an extremely high dilution factor may produce less accurate dilution, while an extremely low dilution factor may provide an increase in the number of subsequent dilution cycles. Such an extremely high or low dilution factor is therefore not preferable. In the assay of the present invention in which the order-of-magnitude accuracy is required, it is preferable to serially dilute a sample by 10-fold for ease of calculation of cell count. In this case, for example, 1 part of a sample and 9 parts of a diluent solution are combined to give a 10-fold dilution. This dilution procedure can be repeated to give a series of 10-fold dilutions including 10-fold dilution, 100-fold dilution, and 1,000-fold dilution etc.

In the second step of the present invention, each dilution from the first step is cultured. As used herein, the term "culture" or "culturing" means that living cells are allowed to be grown experimentally in a growth medium for living cells under controlled external conditions including temperature, oxygen concentration, and culture period etc. Once target living cells and a growth medium to be used are specified, those skilled in the art will readily determine external conditions suitable for cell culture. In the case of bacterial cells, for example, a dilution containing bacterial cells diluted with a growth medium for living cells in a test tube is preferably cultured at 30 °C for 6 hours while shaking at 100 rpm. Depending on target living cells, culture conditions may be selected from the following ranges as appropriate: a culture temperature from 5 to 80 ° C, a shaking condition from static culture to shaking culture at 500 rpm, and a culture period from 1 to 120 hours.

In the present invention, background ATP present in a dilution can cause a decrease in detectability because the presence of living cells is determined based on their intracellular ATP level. Thus, it is desirable to decompose the background ATP present in a diluent solution and a dilution or cultured dilution, prior to the extraction of intracellular ATP from the living cells. To decompose the background ATP, for example, a reagent containing ATPase as an active ingredient (hereinafter, ATP-eliminating agent) may be used. Illustrative examples of ATPase include adenosine phosphate deaminase, apyrase, alkaline phosphatase, acid phosphatase, hexokinase, and phosphodiesterase. These enzymes may be used alone or in combination. After decomposing the background ATP, it is desirable to remove or deactivate the ATP-eliminating agent in a dilution. The ATP-eliminating agent may also be deactivated by treatment with its inhibitor, including coformycine against adenosine phosphate deaminase, actinomycin D against alkaline phosphatase, xylose against hexokinase, and xanthine against phosphodiesterase. In addition to these inhibitors, appropriate physical conditions (e.g., heat, pH, ionic strength) may also be used for deactivation.

In the present invention, the decomposition of background ATP may be achieved by a method in which a diluent solution pre-treated with the ATP-eliminating agent to decompose ATP is used as a diluent solution for the first step, or by a method in which the background ATP is decomposed with the ATP-eliminating agent during or after the culture in the second step. These methods may be used in combination.

The steps of the present invention may be combined with cell collection using a filter in order to further improve the detectability. For example, each dilution from the first step may be filtrated through a filter to collect cells, which may then be cultured in the second step. Alternatively, each cultured dilution from the second step may be filtrated through a filter to collect cells, which may then be used for intracellular ATP detection in the third step.

To detect intracellular ATP in the third step of the present invention, the intracellular ATP is first extracted from living cells present in a cultured dilution or an aliquot taken therefrom, and the extracted ATP is then assayed for its level. Secondly, the ATP level thus obtained is compared with an ATP level detected in a control sample without living cell (blank value) to determine the presence of living cells. For example, in the below-mentioned ATP detection based on the luciferin-luciferase luminescent reaction method, a sample can be identified to contain living cells when luminescence produced from the sample has an intensity of at least 1.5 times higher than the blank value. However, such a numerical value may be determined appropriately, depending on the types of living cell and diluent solution, and/or how the sample and control are prepared.

The intracellular ATP may be extracted from living cells by heat extraction or by treatment with an ATP-extracting reagent. In the former, the dilution is heated at 50 to 100 °C for 1 to 60 minutes to release the intracellular ATP out of the cells. In the latter, the ATP-extracting reagent is added to the dilution, followed by extraction. As an ATP-extracting reagent, a known reagent may be used, including lysozyme, a mixture of ethanol and ammonia, methanol, ethanol, surfactants (e.g., benzethonium chloride, benzalkonium chloride, Triton X-100), trichloroacetic acid, and perchloric acid. For example, an ATP-extracting reagent packaged with CheckLite 250 Plus commercially available from Kikkoman Corp. may be used. Next, the extracted ATP may be detected by various known ATP detection methods. For example, an ATP-detecting reagent may be added to the dilution after ATP extraction. Any reagent for detecting ATP can be used for this purpose. Specifically, a reagent for detecting ATP based on the luciferin-luciferase luminescent reaction method may be employed. Luciferin and luciferase used in the present invention may be those derived from fireflies such as Genji-firefly (*Luciola cruciata*), Heike-firefly (*Luciola lateralis*), American firefly (*Photinus pyralis*), and glowworm.

In the present invention, luciferase may be a naturally occurring luciferase purified from luminous tissue of organisms mentioned above, a genetically engineered wild-type luciferase, or a mutant luciferase modified by one or more amino acid addition, deletion and substitution in the amino acid sequence for the wild-type luciferase.

In the luciferin-luciferase luminescent reaction method, an ATP-detecting reagent comprising luciferin, luciferase and magnesium (hereinafter, bioluminescent reagent) is first added to the dilution after ATP extraction. Since luminescence is produced through the reaction between the bioluminescent reagent and ATP, its intensity is measured using a luminometer and the like. As a bioluminescent reagent, a bioluminescent reagent packaged with a commercially available reagent, for example, CheckLite 250 Plus (Kikkoman Corp.) may be used. Instead of the above luciferin-luciferase luminescent reaction method, a bioluminescence method modified to include pyruvate orthophosphate dikinase (hereinafter, PPDK luminescence method) can be used in order to further improve the detectability (Japanese Patent Laid-Open Publication No. 9-234099).

In the fourth step, the number of living cells in the original sample is determined based on the detected intracellular ATP level in each cultured dilution. Namely, the number of living cells in the sample and/or cell count with order-of-magnitude accuracy can be calculated from the intensity of luminescence produced, the ATP level obtained therefrom, and the dilution factor used. Simultaneously, a more accurate cell count for the sample may also be calculated using the most probable number table.

An assay reagent kit for counting living cells comprises the diluent solution, ATP-extracting reagent and ATP-detecting reagent mentioned above. ATP-extracting and -detecting reagents may be a commercially available reagent for detecting intracellular ATP based on the luciferin-luciferase luminescent reaction method, for example, CheckLite 250 Plus (Kikkoman Corp.). When the decomposition of background ATP is required for detection with high detectability, the background ATP is decomposed as described above, for example, using a CheckLite ATP Eliminating kit commercially available from Kikkoman Corp., prior to addition of the ATP-extracting agent. The kit may comprise other reagents, in addition to the above diluent solution and reagents. Examples of other reagents include an ATP-eliminating agent or the like.

Further, the living cell counting device according to the present invention, which is used for carrying out the above cell counting method, comprises a device for sample dilution, a culture device for cell growth and a device for ATP detection. The device for sample dilution is used for the serial dilution of a sample in test tubes. A commercially available dilution device, such as System Diluter (distributed by Gunze Sangyo, Inc.), may also be used for this purpose. The dilution device can automatically select any desired dilution condition, including diluent solution volume and serial dilution factor, under the control of a program stored therein. In turn, the culture device for cell growth is used for culturing living cells present in a dilution. Preferably, it can comprise a thermostat and a shaker.

The device for ATP detection comprises an extractor for extracting intracellular ATP and a detector for detecting the extracted ATP. The extractor can automatically extract intracellular ATP at room temperature by adding a predetermined amount of the ATP-extracting agent to a test tube containing the cultured dilution. The detector may be, for example, a luminometer for detecting bioluminescence produced through the luciferin-luciferase luminescent reaction. In this case, a predetermined amount of a luciferin-luciferase luminescent reagent is added to a test tube after ATP extraction to measure the intensity of luminescence produced. A suitable ATP detector, which can be used in the present invention, includes a commercially available detector, Lumitester K-100 (distributed by Seishin Corp.).

The number of living cells in the original sample is calculated from the resulting ATP level in the detection. The living cell counting device of the present invention may incorporate an arithmetic unit having the following calculation program:
(1) a program which calculates cell count from ATP levels and dilution conditions; or
(2) a program which calculates cell count from ATP levels and dilution conditions using the most probable number method based on a statistical probability theory. In addition, the device may also have additional functions including storage, backup and printout of various data.

The device for sample dilution, culture device for cell growth and device for ATP detection according to the present invention may be functionally connected with one another to provide the living cell counting device. Preferably, these three devices may be integrated into a unit system, such that the number of living cells in a sample can be determined automatically by just loading the sample in the system.

### EXAMPLES

The present invention will be further described in the following examples. These examples are provided for illustrative purposes only, and are not intended to limit the scope of the invention.

### Example 1: Determination of Living Cell Counts using Standard Bacterial Strains

As standard bacterial strains, *Escherichia coli* ATCC 25922 (hereinafter, Strain E) and *Bacillus subtilis* ATCC 9372 (hereinafter, Strain B) were used. Each of these two standard bacterial strains was inoculated into 3 ml of nutrient broth liquid medium and cultured overnight at 35 °C. Each resulting culture was appropriately diluted with sterilized physiological saline and used as a sample for the living cell counting mentioned below. A sample containing more living cells was designated Sample 1, while a sample containing less living cells was designated Sample 2.

### (1) Inventive Method

10 ml of ATP-eliminating agent such as CheckLite ATP Eliminating Kit (Kikkoman Corp.), which had been sterilized by filtration, was added to 90 ml of autoclaved fluid thioglycolate medium II (Eiken Chemical Co., Ltd.) to give a culture medium for living cells containing no ATP, which was then used as a diluent solution. 1 ml of Sample 1 was added to and stirred well with 9 ml of the diluent solution to produce a 10-fold dilution. Next, 1 ml of the 10-fold dilution was added to and stirred with 9 ml of the diluent solution to produce a 100-fold dilution. Finally, the same procedure was repeated to give a series of 10-fold dilutions, including 10-fold, 100-fold, 1,000-fold, 10,000-fold or more dilutions. Each dilution thus obtained was cultured at 35 °C for 6 hours. After culturing, 0.1 ml of each cultured dilution was transferred to a cuvette, followed by intracellular ATP extraction and bioluminescent reaction using CheckLite 250 Plus (Kikkoman Corp.). The intensity of bioluminescence resulting from the intracellular ATP was measured using a Lumitester K-100 (distributed by Kikkoman Corp.). The intensity of luminescence produced from the diluent solution alone (0.1 ml) was used as a blank value. When luminescence produced from a cultured dilution had an intensity of at least 1.5 times higher than the blank value, the cultured dilution was identified to contain living cells. Out of the dilutions identified to contain living cells, the dilution with a maximum dilution factor was chosen to calculate living cell count for the original sample from the dilution factor value.

### (2) Comparative Method 1

Sterilized physiological saline was used as a diluent solution. Each sample was serially diluted to give a series of 10-fold dilutions. 100 µl of each dilution was applied on a plate count agar (Eiken Chemical Co., Ltd.) in a plastic schale and then cultured at 35 °C for 2 days. Colonies formed on the plate were counted to calculate living cell count for the original sample from the dilution factor value.

### (3) Comparative Method 2

1.0 ml of each sample was transferred to a cuvette and reacted with 0.1 ml of ATP-eliminating agent at room temperature for 30 minutes. 0.1 ml of the reacted sample was transferred to a cuvette, followed by intracellular ATP detection using CheckLite 250 Plus (Kikkoman Corp.) and a Lumitester K-100 (distributed by Kikkoman Corp.). The measured intensity of luminescence resulting from the intracellular ATP was converted into living cell count using the calibration curve shown in Figure 1.

### (4) Results

(a) In the case of Strain E:
(i) The results obtained by the inventive method are shown in Table 1.

**Table 1**

| Dilution factor | Sample 1 | | Sample 2 | |
|---|---|---|---|---|
| | Intensity (RLU) | Growth | Intensity (RLU) | Growth |
| 10 | 284404 | Yes | 3095 | Yes |
| 100 | 27898 | Yes | 255 | Yes |
| 1,000 | 2467 | Yes | 10 | No |
| 10,000 | 366 | Yes | 11 | No |
| 100,000 | 10 | No | 10 | No |
| 1,000,000 | 9 | No | 9 | No |
| Blank value | 10 | No | 10 | No |
| Living cell count for Sample 1 (order of magnitude): 10⁴ CFU/ml | | | | |
| Living cell count for Sample 2 (order of magnitude): 10² CFU/ml | | | | |

(ii) The results obtained by comparative method 1 are shown in Table 2.

**Table 2**

| Sample | Living cell count |
|---|---|
| Sample 1 | 4.5 × 10⁴ CFU/ml |
| Sample 2 | 4.5 x 10² CFU/ml |

(iii) The results obtained by comparative method 2 are shown in Table 3.

**Table 3**

| Sample | Intensity | Living cell count |
|---|---|---|
| Sample 1 | 130 RLU | 1.8 × 10⁴ CFU/ml |
| Sample 2 | 4 RLU | uncountable |
| Blank | 5 RLU | |

(b) In the case of Strain B:
(i) The results obtained by the inventive method are shown in Table 4.

**Table 4**

| Dilution factor | Sample 1 | | Sample 2 | |
|---|---|---|---|---|
| | Intensity (RLU) | Growth | Intensity (RLU) | Growth |
| 10 | 77422 | Yes | 306 | Yes |
| 100 | 2249 | Yes | 53 | Yes |
| 1,000 | 181 | Yes | 12 | No |
| 10,000 | 22 | Yes | 11 | No |
| 100,000 | 11 | No | 9 | No |
| 1,000,000 | 9 | No | 10 | No |
| Blank value | 10 | No | 10 | No |
| Living cell count for Sample 1 (order of magnitude): 10⁴ CFU/ml | | | | |
| Living cell count for Sample 2 (order of magnitude): 10² CFU/ml | | | | |

(ii) The results obtained by comparative method 1 are shown in Table 5.

**Table 5**

| Sample | Living cell count |
|---|---|
| Sample 1 | 4.4 × 10⁴ CFU/ml |
| Sample 2 | 4.4 × 10² CFU/ml |

(iii) The results obtained by comparative method 2 are shown in Table 6.

**Table 6**

| Sample | Intensity | Living cell count |
|---|---|---|
| Sample 1 | 560 RLU | 1.8 × 10⁴ CFU/ml |
| Sample 2 | 6 RLU | uncountable |
| Blank | 5 RLU | |

Tables 1, 2, 4 and 5 showed that all living cell counts for Samples 1 and 2 determined by the inventive method and comparative method 1 were of the same order of magnitude in both Strains E and B. As shown in Tables 3 and 6, on the other hand, in both Strains E and B, only living cell counts for Sample 1 (containing more cells) determined by comparative method 2 using the calibration curve shown in Figure 1 were of the same order of magnitude as those determined by the inventive method and comparative method 1. With regard to Sample 2, both standard bacterial strains were substantially uncountable by comparative method 2 because they had cell counts less than 10³ CFU/ml and thus an intensity equal to the blank value.

These results indicate that any sample containing more or less cells, including those uncountable by comparative method 2 (less than 10³ CFU/ml), can be counted with order-of-magnitude accuracy in a short time by the inventive method.

### Example 2: Determination of Contaminant (Living Cell) Counts in Cut Vegetables

Three types of commercially available cut vegetables were purchased. 100 ml of sterilized physiological saline was added to 10g of each cut vegetable, followed by stomaching treatment using a Stomacher (distributed by Organo Corporation). The stomached solution thus obtained was used as a sample.

### (1) Inventive Method

The above samples were used and assayed according to the inventive method described in Example 1, except for a culture temperature of 30 ° C and a culture period of 8 hours.

### (2) Comparative Method 1

The above samples were used and assayed according to comparative method 1 described in Example 1, except for a culture temperature of 30 °C and a culture period of 2 days.

### (3) Comparative Method 2

The above samples were used and assayed according to comparative method 2 described in Example 1, except that a cell-free sample prepared by filtration through a 0.22 µm filter was similarly assayed to obtain its luminescence intensity as a blank value.

### (4) Results

(i) The results obtained by the inventive method are shown in Table 7.

**Table 7**

| Dilution factor | Sample 1 | | Sample 2 | | Sample 3 | |
|---|---|---|---|---|---|---|
| | Intensity (RLU) | Growth | Intensity (RLU) | Growth | Intensity (RLU) | Growth |
| 10 | 14381 | Yes | Out of range | Yes | 92932 | Yes |
| 100 | 1820 | Yes | 536332 | Yes | 7791 | Yes |
| 1,000 | 180 | Yes | 57266 | Yes | 634 | Yes |
| 10,000 | 80 | Yes | 6782 | Yes | 66 | Yes |
| 100,000 | 10 | No | 986 | Yes | 45 | Yes |
| 1,000,000 | 9 | No | 101 | Yes | 10 | No |
| 10,000,000 | 11 | No | 10 | No | 11 | No |
| 100,000,000 | 12 | No | 12 | No | 12 | No |
| Blank value | 10 | No | 10 | No | 9 | No |
| Living cell count for Sample 1 (order of magnitude): 10⁴ CFU/ml | | | | | | |
| Living cell count for Sample 2 (order of magnitude): 10⁶ CFU/ml | | | | | | |
| Living cell count for Sample 3 (order of magnitude): 10⁵ CFU/ml | | | | | | |

(ii) The results obtained by comparative method 1 are shown in Table 8.

**Table 8**

| Sample | Living cell count |
|---|---|
| Sample 1 | 4.2 × 10⁴ CFU/ml |
| Sample 2 | 4.5 × 10⁶ CFU/ml |
| Sample 3 | 1.3 x 10⁵ CFU/ml |

(iii) The results obtained by comparative method 2 are shown in Table 9.

**Table 9**

| Sample | Intensity | Living cell count |
|---|---|---|
| Sample 1 | 4689 RLU | uncountable |
| Sample 2 | 19028 RLU | 8.0 × 10⁶ CFU/ml |
| Sample 3 | 5538 RLU | uncountable |
| Blank | 4490 RLU | |

In the three samples, all living cell counts determined by the inventive method in a short time (Table 7) and comparative method 1 (Table 8) were of the same order of magnitude. In comparative method 2, as shown in Table 9, Samples 1 and 3 were uncountable because the blank had a high value of 4490 RLU (unit of the intensity) and these two samples had the measured values nearly equal to the blank value. However, Sample 2 containing more contaminants had a significantly high measured value as compared with the blank value, thereby providing the result of the same order of magnitude as that of the inventive method.

These results indicate that any sample having various blank values, including those uncountable by comparative method 2 due to a high blank value, can be counted in a short time by the inventive method. The inventive method can also be used for the determination of contaminant count in foods, such as vegetables, without any problem.

### Example 3: Determination of Contaminant (Living Cell) Counts in Meats

Three types of commercially available meats were purchased. 100 ml of sterilized physiological saline was added to 10g of each meat, followed by stomaching treatment using a Stomacher. The stomached solution thus obtained was used as a sample.

### (1) Inventive Method

The above samples were used and assayed according to the inventive method described in Example 1, except for a culture temperature of 30 °C and a culture period of 8 hours. CheckLite HS Plus (Kikkoman Corp.) was used as a luminescent reagent.

### (2) Comparative Method 1

The above samples were used and assayed according to comparative method 1 described in Example 1, except for a culture temperature of 30 °C and a culture period of 2 days.

### (3) Comparative Method 2

The above samples were used and assayed according to comparative method 2 described in Example 1, except that a cell-free sample prepared by filtration through a 0.22 µm filter was similarly assayed to obtain its luminescence intensity as a blank vale. CheckLite HS Plus (Kikkoman Corp.) was used as a luminescent reagent.

### (4) Results

(i) The results obtained by the inventive method are shown in Table 10.

**Table 10**

| Dilution factor | Sample 1 | | Sample 2 | | Sample 3 | |
|---|---|---|---|---|---|---|
| | Intensity (RLU) | Growth | Intensity (RLU) | Growth | Intensity (RLU) | Growth |
| 10 | 483366 | Yes | 32087 | Yes | Out of range | Yes |
| 100 | 28682 | Yes | 1893 | Yes | 143785 | Yes |
| 1,000 | 723 | Yes | 236 | Yes | 16190 | Yes |
| 10,000 | 56 | Yes | 68 | Yes | 1458 | Yes |
| 100,000 | 25 | No | 26 | No | 208 | Yes |
| 1,000,000 | 24 | No | 23 | No | 59 | Yes |
| 10,000,000 | 25 | No | 25 | No | 25 | No |
| Blank value | 24 | No | 10 | No | 25 | No |
| Living cell count for Sample 1 (order of magnitude): 10⁴ CFU/ml | | | | | | |
| Living cell count for Sample 2 (order of magnitude): 10⁴ CFU/ml | | | | | | |
| Living cell count for Sample 3 (order of magnitude): 10⁶ CFU/ml | | | | | | |

(ii) The results obtained by comparative method 1 are shown in Table 11.

**Table 11**

| Sample | Living cell count |
|---|---|
| Sample 1 | 4.0 × 10⁴ CFU/ml |
| Sample 2 | 1.5 × 10⁴ CFU/ml |
| Sample 3 | 2.8 × 10⁶ CFU/ml |

(iii) The results obtained by comparative method 2 are shown in Table 12.

**Table 12**

| Sample | Intensity | Living cell count |
|---|---|---|
| Sample 1 | 55928 RLU | uncountable |
| Sample 2 | 55160 RLU | uncountable |
| Sample 3 | 57298 RLU | uncountable |
| Blank | 53692 RLU | |

In comparative method 2, as shown in Table 12, all three samples were uncountable because the blank had a very high value of 53692 RLU and all these samples had the measured values nearly equal to the blank value. On the other hand, all living cell counts determined by the inventive method and comparative method 1 were of the same order of magnitude in all three samples. Accordingly, even a sample uncountable by comparative method 2 due to a very high blank value can be counted in a short time by the inventive method. The inventive method is also useful for the determination of contaminant count in foods such as meat.

### INDUSTRIAL APPLICABILITY

The present invention relates to a method for counting living cells in a sample by serially diluting the sample containing living cells with a growth medium for living cells as a diluent solution, culturing the resulting dilution in a short time, and then detecting intracellular ATP. The present invention also relates to an assay reagent kit used for the above method and a living cell counting device.

By using the present invention, the number of living cells in a sample can be determined with high sensitivity in a short time. In foods, particularly perishable foods, it is strongly desirable to count contaminants contained therein in a short time. Accordingly, the present invention can be used preferably for this purpose.

All publications cited herein are incorporated herein by reference in their entirety.

## Claims

1. A method for counting living cells in a sample, which comprises the following steps (i) to (iv):
(i) a first step for serially diluting the sample with a diluent solution;
(ii) a second step for culturing each dilution from the first step;
(iii) a third step for detecting intracellular ATP in each of the cultured dilutions from the second step; and
(iv) a fourth step for calculating the number of living cells in the sample, based on the results of intracellular ATP detection from the third step.

2. The method according to claim 1, wherein the diluent solution used in the first step is a growth medium for living cells.

3. The method according to claim 1, wherein the diluent solution used in the first step is a growth medium for living cells which has been subjected to ATP decomposition.

4. The method according to claim 1, wherein ATP present in the dilution, excluding the intracellular ATP, is decomposed during or after the culture in the second step.

5. The method according to claim 1, wherein the intracellular ATP is detected in the third step using a luciferin-luciferase luminescent reaction.

6. An assay reagent kit used for the method according to claim 1, which comprises the following components (a) to (c):
(a) a diluent solution,
(b) an ATP-extracting reagent, and
(c) an ATP-detecting reagent.

7. A living cell counting device used for the method according to claim 1, which comprises a device for sample dilution, a culture device for cell growth and a device for ATP detection.

8. The living cell counting device according to claim 7, wherein the device for ATP detection measures the intensity of luminescence produced through a luciferin-luciferase luminescent reaction.
